# EUROPEAN PATENT APPLICATION

(11) **EP 3 438 240 A1**
(43) Date of publication of application: **06.02.2019**
(21) Application number: 18186657.5
(22) Date of filing: 31.07.2018
(51) Int. Cl.: C12M 1/107, C02F 3/00, C12M 1/00, C12M 1/33

(54) **BIOGAS SYSTEM FOR GENERATING ELECTRIC POWER**

(30) Priority: 03.08.2017 IT 201700089383
(71) Applicant: Irim S.R.L., 24050 Ghisalba (BG) (IT)
(72) Inventor: GHISLOTTI, Bortolo, 24050 Ghisalba BG (IT)
(74) Representative: Modiano, Micaela Nadia

(57) **Abstract**

A biogas system (1) for generating electric power, comprising a receiving pre-vat (2) for the wastewater, a line (3) for feeding the wastewater from the receiving pre-vat (2) to at least one digester (4), a discharge line (5) designed to discharge the wastewater treated by the at least one digester (4) into at least one storage vat (6), and a cogenerator (7) for generating electric power and thermal energy, which is supplied by means of the biogas generated in the at least one digester (4); the system (1) has, along the supply line (3), heat exchange means (10) adapted to heat the wastewater that passes through said supply line (3), the heat exchange means using thermal energy generated by the cogenerator (7).

## Description

The present invention relates to a biogas system for generating electric power.

The term biogas refers to a mixture of gases, predominantly methane and carbon dioxide, obtained from the anaerobic fermentation (digestion) of various types of organic waste, such as for example zootechnical wastewater or other agroindustrial organic waste.

In recent years, biogas generation systems have become increasingly widespread, since biogas can be burned to generate electric power or used in cogeneration systems.

Biogas systems allow to produce biogas starting from the anaerobic fermentation of wastewater of zootechnical farms (dairy cows and pigs), bioenergy which at the end of the process will be converted into electric and thermal power, thereby adding significant value in economic terms to the farm.

The generation of renewable energy from a biogas system is a real and concrete answer to current pressing environmental demands, which have peaked with the signing of the Kyoto Protocol in December 1997, which came into force on 16 February 2005.

Biogas systems allow to obtain methane from wastewater thanks to anaerobic digestion processes.

Anaerobic digestion is a complex biological process which can be described simply and briefly as follows: a special microbial flora converts, in the absence of oxygen, the selected organic substance into biological gas. The gas is constituted by a mixture to a large extent of methane and carbon dioxide, characterized by a high energy value.

Controlled anaerobic processes are divided according to the following criteria:
- thermal regimen:
   psychrophilia (20 °C) (scarcely used industrially) mesophilia (35-37 °C)
   thermophilia (50 °C and above);
- solid content in the reactor:
   wet process (5-8% dry substance)
   semi-dry process (8-20% dry substance)
   dry process (dry substance > 20%)
- biological step:
   single: the entire microbial chain is kept in a single reactor
   separate: the fermentative hydrolysis steps are separate from the methanogenic step.

The percentage of methane in the biogas varies from a minimum of 50% to a maximum of approximately 75%, as a function of the type of organic substance used and of the process conditions.

In general, the raw materials that can be used for the production of biogas are wastewater, zootechnical residues and biomasses with low lignin content.

Since energy production is integrated directly in agricultural activity, various advantages of the energy-related, environmental and agricultural kind emerge which can be summarized as follows:
- production of energy from a renewable source with global reduction of CO2 emissions;
- improvement of the economy of zootechnical and/or agricultural farms;
- lower emissions of greenhouse gases;
- improvement of the quality of the produced fertilizers;
- economic recycling of wastewater with positive effect on environmental impact;
- reduced olfactory pollution and reduction of the presence of insects;
- improvement of hygienic-sanitary conditions of the facility.

However, known systems are not free from drawbacks.

In particular, the loading of the wastewater from a pre-vat into the digesters can cause a sudden lowering of the temperature of the wastewater during digestion and this inevitably slows the digestion process.

The aim of the present invention is to provide a biogas system that is capable of improving the prior art in one or more of the aspects indicated above.

Within this aim, an object of the invention is to provide a biogas system that is highly reliable, relatively easy to provide and at competitive costs.

This aim, this object and others which will become better apparent hereinafter are achieved by a biogas system according to claim 1, optionally provided with one or more of the characteristics of the dependent claims.

Further characteristics and advantages of the invention will become better apparent from the description of some preferred but not exclusive embodiments of the biogas system according to the invention, illustrated by way of non-limiting example in the accompanying drawings, wherein:

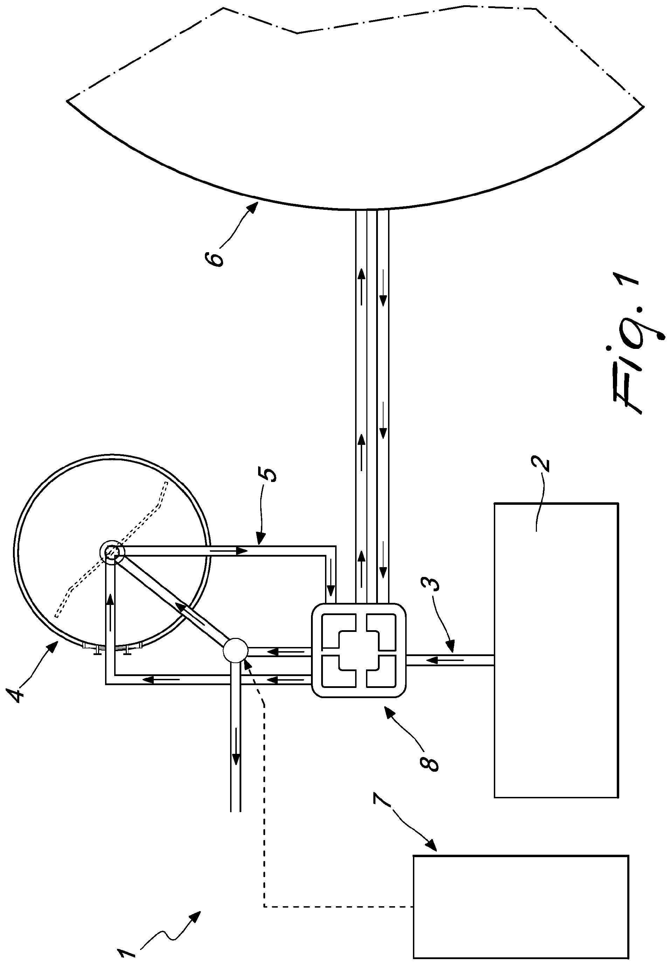

Figure 1 is a schematic view of the system according to the invention;

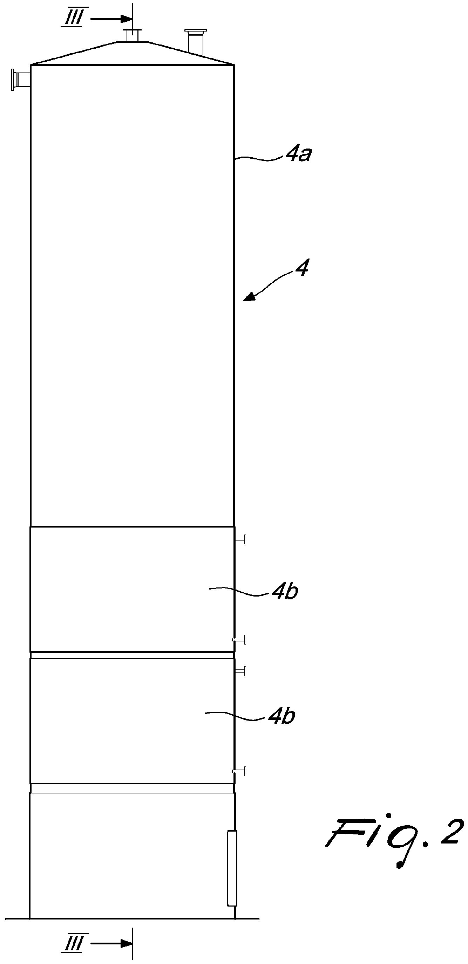

Figure 2 is a lateral elevation view of a digester;

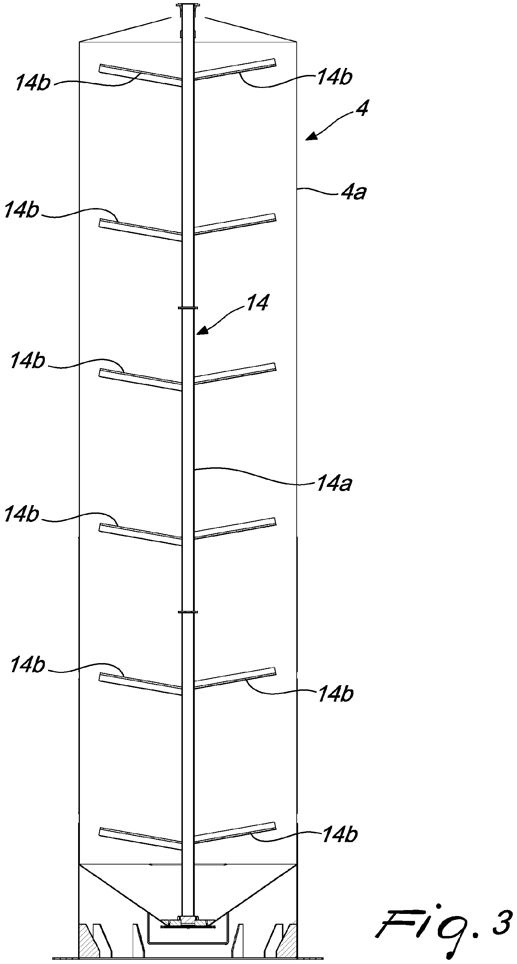

Figure 3 is a sectional view of the digester, taken along the plane of arrangement defined by the line III-III of Figure 2.

With reference to the cited figures, the biogas system for generating electric power according to the invention, generally designated by the reference numeral 1, comprises at least one wastewater receiving pre-vat 2, a line 3 for feeding the wastewater from the receiving pre-vat 2 to at least one digester 4, a discharge line 5 designed to discharge the wastewater treated by the at least one digester 4 into at least one storage vat 6, and a cogenerator 7 for the production of electric power and thermal energy which is supplied by means of the biogas produced in the digester or digesters 4 and stored in the vat 6.

According to the present invention, the system 1 comprises, along the supply line 3, heat exchange means 10 adapted to heat the wastewater that passes through the supply line 3: the heat exchange means use thermal energy produced by the cogenerator 7.

Conveniently, the or each digester 4 comprises a digestion tank defined laterally by a cylindrical side wall 4a.

Specifically, there is at least one heat exchanger arranged substantially at the cylindrical side wall and functionally associated with the cogenerator.

By way of example, the heat exchanger can comprise a so-called ferrule 4b, i.e., an interspace provided inside the cylindrical side wall, which is crossed by water at a temperature that is higher than the ambient temperature and arrives from the cogenerator 7.

Preferably, the or each digestion tank is associated internally with a wastewater mixing device 14.

By way of example, the wastewater mixing device 14 comprises a shaft 14a which is arranged axially to the cylindrical side wall 4a and is associated with a plurality of mixing blades 14b which have a substantially radial extension and are mutually spaced angularly and longitudinally.

Conveniently, the biogas system 1 comprises, along the supply line 3, an assembly 8 for pumping the wastewater from the pre-vat toward the or each digestion tank.

According to a preferred practical embodiment, the pumping assembly 8 is functionally connected to the discharge line.

Conveniently, the pre-vat comprises a device for triturating the wastewater received by it.

Specifically, the pre-vat is associated with wastewater loading means and in particular with means for loading liquid wastewater and the dry fraction.

The trituration device is defined to triturate the dry part so as to render the wastewater pumpable by the pumping assembly 8.

The use of a biogas system 1, according to the invention, is understood evidently from what has been described above.

In practice it has been found that the invention achieves the intended aim and objects.

The invention thus conceived is susceptible of numerous modifications and variations, all of which are within the scope of the appended claims; all the details may further be replaced with other technically equivalent elements.

In practice, the materials used, so long as they are compatible with the specific use, as well as the contingent shapes and dimensions, may be any according to the requirements and the state of the art.

The disclosures in Italian Patent Application no. 102017000089383, from which this application claims priority, are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A biogas system (1) for generating electric power, comprising a receiving pre-vat (2) for the wastewater, a line (3) for feeding said wastewater from said receiving pre-vat (2) to at least one digester (4), a discharge line (5) designed to discharge the wastewater treated by said at least one digester (4) into at least one storage vat (6), and a cogenerator (7) for generating electric power and thermal energy, which is supplied by means of the biogas generated in said at least one digester (4), **characterized in that** it comprises, along said supply line (3), heat exchange means (10) adapted to heat the wastewater that passes through said supply line (3), said heat exchange means using thermal energy generated by said cogenerator (7).

2. The biogas system (1) according to claim 1, **characterized in that** said receiving pre-vat is associated with a device for triturating the wastewater received in said receiving pre-vat.

3. The biogas system (1) according to one or more of the preceding claims, **characterized in that** at least one digester (4) comprises a digestion tank which is formed laterally by a cylindrical side wall, there being at least one heat exchanger associated with said cylindrical side wall and functionally associated with said cogenerator.

4. The biogas system (1) according to one or more of the preceding claims, **characterized in that** said at least one digestion tank is associated internally with a wastewater mixing device.

5. The biogas system (1) according to one or more of the preceding claims, **characterized in that** said heat exchanger is functionally connected to said cogenerator (7).

6. The biogas system (1) according to one or more of the preceding claims, **characterized in that** it comprises, along said supply line (3), an assembly (8) for pumping the wastewater from said pre-vat toward said at least one digestion tank.

7. The biogas system (1) according to one or more of the preceding claims, **characterized in that** said receiving pre-vat is associated with means for loading the liquid wastewater and the dry fraction, said trituration device being designed to triturate the dry fraction so as to render the wastewater pumpable by the pumping assembly (8).

8. The biogas system (1) according to one or more of the preceding claims, **characterized in that** said pumping assembly (8) is functionally connected to said discharge line.

9. The biogas system (1) according to one or more of the preceding claims, **characterized in that** said storage vat forms in an upper region a body for storing the biogas generated in said at least one digester.

10. The biogas system (1) according to one or more of the preceding claims, **characterized in that** said storage vat is provided in an upper region with a net.
